# EUROPEAN PATENT APPLICATION

(11) **EP 4 233 990 A1**
(43) Date of publication of application: **30.08.2023**
(21) Application number: 22382172.9
(22) Date of filing: 28.02.2022
(51) Int. Cl.: A61N 1/04, A61N 1/36, A61B 5/296

(54) **MULTI-PAD ELECTRODE AND SYSTEM FOR ELECTRICAL STIMULATION AND/OR RECORDING COMPRISING PRINTED LOGIC CIRCUITS**

(71) Applicant: Fundación Tecnalia Research & Innovation, 48160 Derio - Bizkaia (ES)
(72) Inventor: Strbac, Matija, BELGRADE (RS); Kostic, Milos, BELGRADE (RS); Isakovic, Milica, BELGRADE (RS); Keller, Thierry, DONOSTIA/SAN SEBASTIÁN (ES); Ersman, Petern Andersson, NORRKÖPING (SE); Torah, Russel, SOUTHAMPTON (GB)
(74) Representative: Balder IP Law, S.L.

(57) **Abstract**

A multi-pad electrode (10, 100, 200, 400) comprising a flexible substrate (11) having a multilayer structure printed thereon, is disclosed. The printed multilayer structure comprises: a logic circuit (25, 125, 225); a plurality of switching elements (135, 235) configured for stimulation or recording; and a plurality of electrode pads (40), each electrode pad (40) being in contact with a respective switching element (135, 235). Each switching element (135, 235) is configured to connect the logic circuit (25, 125, 225) to a respective electrode pad (40), the logic circuit (25, 125, 225) being configured to activate or deactivate each electrode pad (40), by controlling propagation of an electrical signal (signal in/out) to each switching element (135, 235) and therefore to each electrode pad (40) or vice versa. A system for electrical stimulation (1) is also disclosed. It comprises: a multi-pad electrode (10, 100, 200, 400); a stimulation/recording device (50), configured to provide stimulating electrical signals to the multi-pad electrode (10, 100, 200, 400), or recording of electrical signals from active electrode pads; and a controller (60) configured to control the propagation of electrical signals from/to the stimulation/recording device (50) to/from the electrode pads (40) of the multi-pad electrode (10, 100, 200, 400).

## Description

### TECHNICAL FIELD

The present invention relates to the field of wearable electrodes having a plurality of pads, such as wearable electrodes for applications of electrical stimulation and/or recording of electrophysiological signals of the body of a user or parts thereof. It also relates to systems for electrical stimulation and/or recording comprising wearable electrodes.

### STATE OF THE ART

Electrodes are used in various biomedical applications related to electrical stimulation and measuring of electrophysiological signals on the surface of the skin. Electrodes that allow controlled propagation and delivery of electrical pulses are applied, for example, in neural rehabilitation, neuromodulation for tremor suppression and electro-analgesia, cosmetics, sports, wound healing and drug delivery, among others. Electrodes for electrophysiological recording are essential in diagnostics and human-machine interfacing. Most notably, electrical activity of the muscles (electromyography, EMG) is used for controlling various devices, ranging from prosthetic limbs with multiple degrees of freedom to consumer devices, such as computers, video games, home appliances and drones.

The shape and placement of electrodes, when used for muscle activation through the delivery of electrical stimulation, influence muscle selectivity and recruitment. In turn, the shape and placement of electrodes, when used for sensing, influence the quality of the signal content. These two parameters (shape and placement) are of key importance for application of biomedical electrodes in both stimulation and recording, and electrode shape selection and proper placement is one of the main impediments in practical applications.

Multi-pad electrodes have been proven as an effective approach to achieve dynamic change of shape, size and configuration of the electrodes. Multi-pad systems are often used to address naturally occurring variability in biological systems. Multi-pad electrodes comprise a matrix of independent pads that can be grouped into subsets, named virtual electrodes. Independent pads of which a multi-pad electrode is made can be activated in a sequence or selected simultaneously to form a single virtual electrode with all pads on the same potential. By allowing a programmable interface to alter the active electrode surface and position, multi-pad electrodes provide a personalized solution for each patient (in general, user).

Several transcutaneous electro-stimulation devices comprising multi-pad electrodes have already been disclosed. For example, EP2519312B1 discloses an apparatus for external activation of paralyzed body parts by electrostimulation of peripheral nerves comprising a soft apparel provided with multi-pad electrodes on one side and electrode activators on the other side. EP3154627B1 discloses a functional electrical stimulation device for correction of drop-foot disorders, provided with a plurality of multi-pad electrodes configured to be placed on a paretic leg of a user. US6301500B1 discloses an electro-stimulation apparatus comprising an electrode system for measuring local electrical impedance. The electrode system has a plurality of electrode pads.

Devices based on multi-pad electrodes, for both electrical stimulation and EMG recording, have also been disclosed. For example, US8145318B2 discloses an apparatus for electrical stimulation of muscle tissues comprising an electrode system having an electrode array with a plurality of electrode pads for electrical stimulation and a recording sensor for sensing properties of the muscle tissue. Activation and deactivation of the electrode pads is controlled from a control unit separated from the electrode system. US8285381 B2 discloses a system form neuromuscular electrical stimulation of muscle tissue, having a stimulation assembly and a control unit. The stimulation assembly is comprised of a plurality of electrodes for providing electrical stimulation and several sensors for monitoring the remote effects of electrical stimulation from the electrodes on a selected muscle-tendon region of the body. The control unit provides stimulation signals to the plurality of electrodes and receives feedback from the sensors.

However, in conventional electro-stimulation devices, logic circuits for controlling operation of the electrode pads are located in a separate part from the stimulation structure (for example, in a stimulating/recording device). This causes that each pad of the multi-pad electrode has to be connected to the stimulating/recording device through a direct lead or connection (physical line). Therefore, increasing the number of pads (i.e. contact points with the user's skin) results in an exponential rise in complexity and, in practice, limits the spatial resolution (the number of individual pads forming the multi-pad electrode). In other words, the increase in the number of physical connections needed for interfacing the electrode and the recording/stimulation device, caused by an increase in the number of pads, is a major limitation in the state-of-the-art systems, as the number of pads determines the spatial resolution and/or the number of functions that can be effectively managed by the multi-pad system. In summary, while multi-pad systems can provide a cost effective "mass customisable" approach via software to adapt the electrode array to a specific user, their effectiveness is highly dependent on the number of pads that can be used. This is because both resolution and covered area are essential when aiming for a one-size-fits-all solution. Therefore, the practical limitation in the number of pads that can be physically connected means that the state-of-the-art devices and systems cannot fully address new challenges, such as the provision of a one-size electrode matrix suitable to accommodate different body dimensions. This is because of the linear relation between the number of electrodes and number of external connections required in state-of-the-art devices.

Therefore, there is a need to develop new stimulation and recording systems based on multi-pad electrodes, which overcome the drawbacks of conventional ones.

### DESCRIPTION OF THE INVENTION

The present invention overcomes the drawbacks of conventional stimulation and recording systems by providing a multi-pad electrode having a multilayer structure printed on a flexible substrate. In the new multi-pad electrode, active electronic components for pad selection and configuration, which in conventional stimulation and/or recording systems are located in a control unit external to (separated from) the multi-pad electrode, are migrated to the multilayer structure of the multi-pad electrode, which incorporates printed logic circuits, thus enabling the implementation of a greater number of pads and increased usability of the multi-pad technology. A digital circuit (also referred to as logic circuit) is implemented in the printed electronics (multilayer structure), which allows individual electrode pads to be connected in arbitrary configurations to the output leads of the multi-pad electrode. The digital circuit may operate as a switching matrix or switching network. Therefore, the pads can be flexibly organized into several virtual electrodes of arbitrary position, shape and size, which can then be connected to any standard recording and/or stimulation system (such as standard multichannel or single-channel recording and/or stimulation systems). The digital circuit, which functionally belongs to a logic layer implemented in the multilayer structure, can be implemented in different manners. The digital circuit or logic circuit is also referred to as switching logic circuit along this text.

In this text, the term "layer" when used in the expression "functional layer" refers to elements, such as circuits, involved in a certain functional role. In turn, the term "multilayer" (used for example in the expression "multilayer structure") refers to the manufacturing process with which the structure of the electrode is made, in particular to the printing process in which a plurality of printing passes, "layers" or depositions of printing material is required, so that a "multilayer structure" is obtained.

The digital circuit may be implemented as a sequential network, such as a shift register. Alternatively, it may be implemented as a combination network, such as a decoder or a demultiplexer. This provides a paradigm shift in the design of multi-pad recording and stimulation systems. The new paradigm allows that any change in the number of pads forming a multi-pad electrode does not necessarily require a change in the connection and the number of leads connecting the multi-pad electrode to any external electronic system. Therefore, the number of electrode pads can be increased to an arbitrary number while maintaining the same interface, which is of great practical value.

Thus, a new smart multi-pad electrode for transcutaneous delivery of electrical stimuli and/or transcutaneous recording of electrophysiological signals is provided. In the new multi-pad electrode, the switching logic circuit is directly located in the multi-pad electrode, within the multilayer structure, instead of in a separate or peripheral electronic device for stimulation and/or recording, as is the case in conventional devices. The new multi-pad electrode is compatible with arbitrary single- and multi-channel stimulation or recording electronic systems with standardized outputs (i.e. digital outputs). Such standardized outputs will provide the control signals for the configuration of the proposed electrode.

The proposed multi-pad electrode is envisioned in an arrangement of a plurality of electrode pads, such as four or more electrode pads, that can be activated or deactivated (for example independently activated or deactivated) by the digital circuit to form a contact surface with optimized shape and positioning for the intended use.

The multilayer structure of which the multi-pad electrode is made, comprises several functional layers deposited (such as printed) on a flexible substrate. One of these layers is a logic layer implementing the digital circuit or logic circuit of the multilayer structure. The logic layer is preferably implemented as a switching network, such as a combination network or a sequential network (or a combination of both), for controlling signal propagation from a connector (interface with external electronic equipment) to the printed electrode pads. This control is performed via specific switching elements which form the application-specific layer deposited between the logic layer and the layer of pads. As introduced previously, functional layers are not related to the printing process, meaning that the logic layer and the application-specific layer, as well as the pad layer, can also be implemented to share the printable layers of the multilayer printed structure that forms the smart electrode. A switching element connects the logic layer to a respective electrode pad. The digital circuit of the logic layer can be based on a plurality of printed logic switching elements forming a combination network or a sequential network. The application-specific layer is formed by one or multiple application specific switching elements that can (but not necessarily) be different from logic switching elements. The switching elements (that may belong to an application-specific layer or to the logic layer) may be implemented by transistors. In a particular embodiment, they may be implemented by organic electrochemical transistors (OECTs), which are compatible with the printing production processes and have the ability to support significant current throughput at low voltages. Depending on the intended use, the application-specific switching elements, preferably implemented by transistors, such as OECTs, can either act as switches or as amplifiers. For example, they can act as switches for routing a stimulation current from an external stimulator, or they can be used as switches also in the recording application, such that EMG signals are blocked in some electrodes, but allowed to propagate to the connector in the electrodes selected by the logic circuit. They can also amplify the electrophysiological signal arriving at a pad from the body part of the user. In any case they relay the signal between the pads in contact with the human and an electrode connector, providing an interface between said electrode and an external electronic device. The pads and optionally the connector are on the same carrier, and together with the logic layer and with an application specific layer, if present, constitute the proposed electrode. Additionally, the electrode may include an interface layer on top of the pad layer, which is specially designed to ensure stable contact between the electrode pads and the skin of the wearer.

The proposed multi-pad electrode can be produced by deposition of conductive, dielectric and electrochemically active elements or materials onto a flexible substrate used as electrode carrier. More specifically, the deposition process can be multi-layer printing of transferable elements or materials onto the flexible substrate. Depending on the physical and chemical characteristics of the materials used, suitable printing techniques may be screen printing, ink jet printing, aerosol jet printing, flexographic printing, offset printing, thermal transfer printing, or any combination thereof. Non-limiting examples of electrochemically active materials that may be used are: Poly(3,4-ethylenedioxythiophene) doped with poly(styrenesulfonic acid) (PEDOT:PSS), polythiophenes, polyaniline, polypyrrole, and PEDOT deposited through vapor phase polymerization on a pre-deposited oxidant, and/or a combination thereof. These materials can for example be used when OECTs are employed, because they serve as the OECT channel and/or the OECT gate electrode. Non-limiting examples of conductive materials are: electrically conducting polymers, metal, conducting carbon, titanium, platinum, graphite, graphene, noble metals and inert metals or combinations of these or other electron conductive materials. Non-limiting examples of dielectric materials are resins, such as UV curable resins, e.g. ink formulations based on free radical curing acrylic compounds. The materials used are conventional and are out of the scope of the present invention.

The flexible substrate (that is to say, the substrate acting as carrier or electrode carrier) on which the multilayer structure is printed, can be made of different materials. In a possible embodiment, the carrier is a flexible polymer film which can be made of polyethylene terephthalate (PET), thermoplastic polyurethane (TPU), polycarbonate (PC), polystyrene (PS) or a similar organic material. In another possible embodiment, the carrier is a textile material comprising a woven or knitted structure capable to stretch, flex and shear, thus providing compliant breathable and adherent solution for the electrode substrate, with unique drape properties and adherence to the skin.

However, textile materials pose additional challenges when used as a carrier in multilayer structures where high precision deposition is needed, as is the case in multilayer printed logic circuits. One challenge is adhesion, which is inversely correlated to the smoothness of the yarn; another challenge is the smoothness of the overall surface, as protruding fibres (pils) or a rough weave structure can be too uneven to allow deposition of finely pitched complex structures, such as OECTs. In the current case, micrometer-size elements are obtained by depositing multiple layers (printing passes) of material; all these layers need to align with a margin of error on the same scale as the "hills and valleys" that form the surface of the textile carriers. This makes the production of these elements through direct deposition on a textile carrier unfeasible. The invention addresses this issue and, in one embodiment, the smart electrode is produced by first treating the textile carrier by deposition of a primer layer, which ensures good adhesion to the textile and sufficient smoothness of the surface to enable subsequent deposition of the remaining elements of the smart electrode. This primer layer may be based on different elastomers, such as thermoplastic polyurethane (TPU), polyester, polyimide, PDMS. In embodiments of the invention, the layers are printed in sequence such that the first layer provides mechanical adhesion to the textile by being absorbed into the yarns with some smoothing of the surface via compression of any loose fibres. This layer is then cured, with subsequent layers printed and cured on top of this, filling in the weave structure to provide a progressively smoother surface on which to print the following electronic layers. The number of layers required depends, among others, on the roughness of the base fabric. In embodiments of the invention, the primer layer is only deposited on the area of the fabric where the subsequent electrode layers (that is to say, all other layers to be printed, including pads, switching circuitry, etc.) are to be printed, thus maintaining the original properties of the fabric across the remaining area away from the electrode.

Regarding the major limitation in the state-of-the-art systems, which is the number of physical connections needed for interfacing the electrode and the recording/stimulation device, in the present invention the number of pads that can be actively controlled can be increased by an order of magnitude, or can even be arbitrarily large, with respect to state-of-the-art systems. In the claimed invention, the logic circuits for signal routing or switching from the stimulation/recording device to the selected electrode pad -or in the other direction, that is to say, from the selected electrode pad to the stimulation/recording device- are migrated from the stimulation/recording device to the smart electrode, which greatly reduces the number of connections needed in the electrode interface of the device.

In a first aspect of the invention, a multi-pad electrode is provided. The multi-pad electrode comprises a flexible substrate having a multilayer structure printed thereon. The multilayer structure comprises: a logic circuit, a plurality of switching elements (application-specific switching elements) configured for stimulation or recording, and a plurality of electrode pads, each electrode pad being in contact with a respective application-specific switching element. Each application-specific switching element is configured to connect the logic circuit to a respective electrode pad, the logic circuit being configured to activate or deactivate each electrode pad, by controlling propagation of an electrical signal to each switching element and therefore to each electrode pad or vice versa (by controlling propagation of an electrical signal captured by each electrode pad to a switching element and therefore to the logic circuit). For example, the logic circuit is configured to activate or deactivate each electrode pad independently from the activation or deactivation of the other electrode pads.

In embodiments of the invention, the multi-pad electrode further comprises an interface layer in contact with the plurality of electrode pads, the interface layer being an interface configured to ensure stable physical contact between the electrode pads and the skin of the user wearing the multi-pad electrode.

In embodiments of the invention, the flexible substrate is made of a flexible polymer film.

In embodiments of the invention, the flexible substrate is made of a textile material. The textile material of the flexible substrate can comprise an elastomeric material deposited thereon. In a particular embodiment, the elastomeric material deposited on the textile material is a thermoplastic polyurethane (TPU).

In embodiments of the invention, the plurality of switching elements is made of transistors, preferably organic electrochemical transistors.

In embodiments of the invention, the logic circuit is based on transistors, preferably organic electrochemical transistors.

In embodiments of the invention, the multilayer structure printed on the flexible substrate is printed using one of the following printing techniques: screen printing, ink jet printing, aerosol jet printing, flexographic printing, offset printing, thermal transfer printing, or any combination thereof.

In embodiments of the invention, the logic circuit is implemented as a combination network in the form of a demultiplexing unit or a decoder unit configured to allow activation or deactivation of one of 2^{N} electrode pads based on N control input signals. In other words, in these embodiments, the combination network in the logic layer of the electrode can be a demultiplexing unit that allows activation or deactivation of one of 2^{N} electrode pads based on N control input signals. The logic layer may comprise a plurality of combination networks, that is to say, a plurality of demultiplexing units. This limits the functionality of the electrode to the selection of only one active pad, per demultiplexing unit or decoder unit implemented, but requires a simple logic circuit with a low number of components, which introduces proportionally lower latency. In this configuration, each demultiplexing unit or decoder unit with accompanying pads, can be seen as a functional kernel that allows a single channel to be routed to one of N pads implemented in the kernel. In multichannel solutions, these kernels can be stacked to allow post-placement configurability of electrode position for each channel, but without the possibility to change the active pad size or shape. Therefore, this configuration is well suited for applications where a low number of channels is used, but their position on the skin surface should be adjusted often, for example to address dynamic changes in the underlying tissue. An example is neuromuscular stimulation of large muscle groups, like quadriceps, where the therapists often manually move the electrodes disposed on the skin over the muscle surface to induce recruitment of a larger portion of the muscle and avoid early onset of fatigue. Another example is a recording application in which dynamic electrode selection is used to locate a hot spot of muscle activity by probing different pads across the skin, either while the subject is static (calibration recording) or even during movement. The latter could be relevant to track a hot spot that is shifting due to the movement of the muscles below the electrode (e.g., recording electrical signals from a muscle in the forearm while the subject moves the arm).

In embodiments of the invention, the logic circuit is implemented as a shift register configured to allow simultaneous selection of from zero (in practice from one) to M electrode pads based on clock and data signal inputs, M being the total number of electrode pads. [In other words, in these embodiments, the digital circuit in the logic layer of the electrode is a shift register that allows simultaneous activation of one to M electrode pads based on clock and data input signals, where M is the total number of electrode pads. This configuration may be suitable for applications in which it might be required to adjust both the shape and the position of the electrode, e.g. when selective stimulation of complex muscle structures is required, and the solution should fit persons of different sizes, where the same pads can cover different muscle structures depending on the length or girth of the body segment. This is the case of functional electrical stimulation for grasping, where the forearm size, as well as the anatomy of underlying muscles (especially in case of patients), can drastically vary depending on the person's height, weight and constitution. The implementation of this logic circuit requires more complex structures and introduces higher latency in switching, but allows a greater degree of flexibility. Furthermore, when the configuration is being changed the latency is induced by the relatively slow switching characteristics of the switching elements, such as OECTs, in this case in part owing to the requirement of ion diffusion throughout the bulk of the active OECT channel material, which manifests as a ramp transition, rather than a step. Such behaviour is not ideal in all biomedical applications, but in stimulation applications it may lead to a desired physiological-like muscle activation, if the current propagation control is well implemented.

In embodiments of the invention, the logic circuit is implemented with at least one demultiplexing unit or decoder unit and at least one shift register configured to allow simultaneous selection of an arbitrary number of electrode pads based on the combination of control, clock and data input signals. In other words, the logic layer of the smart electrode is implemented by a stack of layers comprising demultiplexing units or decoder units and shift registers. This is the most complex and flexible approach. This multilayer structure allows simultaneous activation of one, or multiple, of the total of M electrode pads based on the combination of control, clock and data input signals. This allows a structure where the number of electrode pads is not a function of the number of input signals. In other words, in such implementation the same physical interface could be used to drive electrodes with an arbitrary number of pads, allowing for unlimited scalability. To address the practical issues of latency and adaptability, in the logic layer of this embodiment, the demultiplexing units and the shift registers have different purposes. Each shift register is used to select a set of electrode pads, which form a virtual electrode, while demultiplexing units or decoder units are subsequently used to dynamically switch previously defined virtual electrodes. In other words, in this case, the at least one shift register can be configured to select a set of electrode pads, thereby forming virtual electrodes, while the at least one demultiplexing unit or decoder unit can be configured to dynamically switch previously defined virtual electrodes. This approach is particularly flexible because it combines the flexibility of shift registers (which are on the other hand slow) with the fastness of demultiplexing units or decoder units (which are on the other hand simple). Therefore, shift registers are used to make complex virtual electrodes, which is done rarely, e.g. once per session, and then it is switched between the pre-defined virtual electrodes with a decoder (or demultiplexer) during the use. This is done often, e.g. a few times in a second.

The disclosed invention addresses both recording and stimulation applications of biomedical electrodes, i.e. an application when the electrical signal is acquired from the body or transmitted to the body, respectively. The disclosed multilayer approach allows for the implementation of these two application domains (recording or stimulating) to differ only in one printing layer.

In embodiments of the invention, in which the multi-pad electrode is envisioned for applications considering acquisition, application-specific switches connecting the electrode pads to the recording channels (these elements being, for example, organic electrochemical transistors), are specifically designed and configured to provide amplification of the input signal.

In embodiments of the invention, in which the multi-pad electrode is envisioned for applications considering electrical stimulation, application-specific switches connecting the electrode pads to the stimulation channels (these elements being, for example, organic electrochemical transistors), are specifically designed and configured to allow high voltages and high current throughput required from the output signal.

In a second aspect of the invention, a system for functional electrical stimulation and/or recording is provided. The system comprises: a multi-pad electrode according to the first aspect of the invention; a stimulation/recording device, configured to provide stimulating electrical signals to the multi-pad electrode or recording electrical signals from active electrode pads; and a controller configured to control the propagation of electrical signals from/to the stimulation/recording device to/from the electrode pads of the multi-pad electrode.

In embodiments of the invention, when the system is configured to provide stimulating electrical signals to the multi-pad electrode, the stimulating electrical signal is provided as a biphasic electrical signal, wherein the negative polarity of said biphasic electrical signal precedes the positive polarity of said biphasic electrical signal.

Additional advantages and features of the invention will become apparent from the detailed description that follows and will be particularly pointed out in the appended claims.

### BRIEF DESCRIPTION OF THE DRAWINGS

To complete the description and in order to provide for a better understanding of the invention, a set of drawings is provided. Said drawings form an integral part of the description and illustrate an embodiment of the invention, which should not be interpreted as restricting the scope of the invention, but just as an example of how the invention can be carried out. The drawings comprise the following figures:
Figure 1 is a schematic representation of a conventional system for electrical stimulation and/or recording.
Figure 2 is a schematic representation of a system for electrical stimulation and/or recording according to embodiments of the invention. The system includes a multi-pad electrode, a stimulator/recorder and a controller.
Figure 3 illustrates a cross-sectional view of a multi-pad electrode according to embodiments of the invention.
Figure 4 illustrates a multi-pad electrode according to an embodiment of the invention, in which the logic circuit of the multi-pad electrode is implemented as a combination network, i.e. a demultiplexer.
Figure 5 illustrates a multi-pad electrode according to an embodiment of the invention, in which the logic circuit of the multi-pad electrode is implemented as a sequential network, i.e. a shift register.

### DESCRIPTION OF A WAY OF CARRYING OUT THE INVENTION

The following description is not to be taken in a limiting sense but is given solely for the purpose of describing the broad principles of the invention. Next embodiments of the invention will be described by way of example, with reference to the above-mentioned drawings, showing apparatuses and results according to the invention.

Next, different embodiments of multi-pad electrodes and systems for electrical stimulation and/or recording, according to the invention, are disclosed.

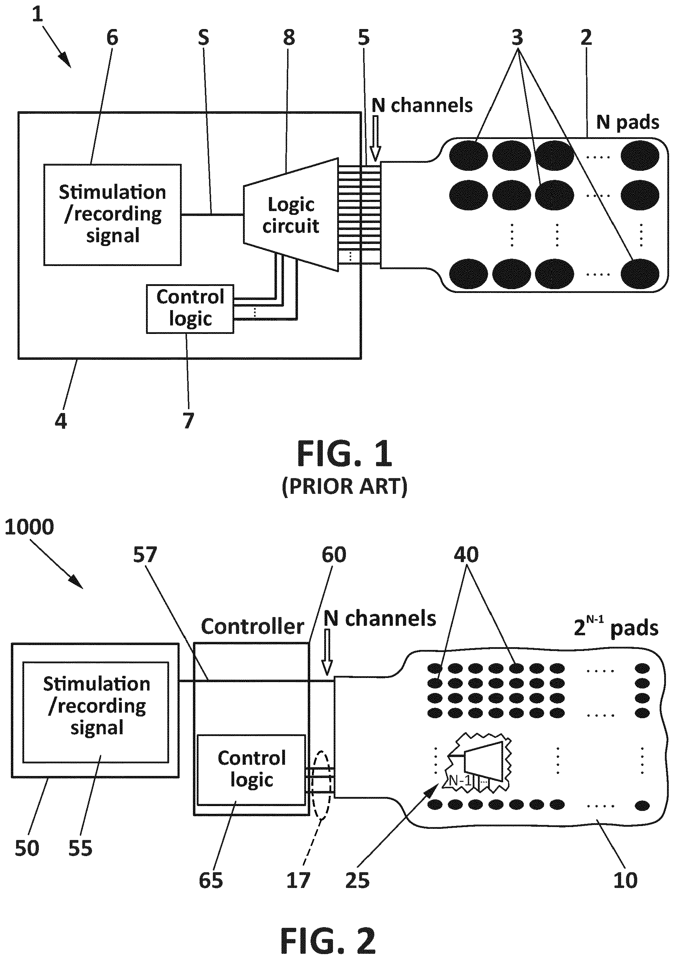

Figure 1 shows a conventional system 1 for electrical stimulation and/or recording. It comprises an electrode 2 (multi-pad electrode), usually made of a flexible substrate. Electrode 2 comprises a plurality of electrode pads 3. Electrode pads 3 may be distributed in an arbitrary configuration. In the shown electrode 2, there are N electrode pads 3 forming a matrix or array of pads, evenly distributed in rows and columns along the surface of the flexible substrate. System 1 also comprises a custom-made (ad hoc) device for stimulation and/or recording (from now on, a stimulator/recorder) 4. N channels 5 connect the stimulator/recorder 4 with the N pads 3 of the electrode 2. In other words, each pad 3 of the N pads of the electrode 2 is connected to the stimulator/recorder 4 by means of a dedicated channel 5 of a set of N channels. These N channels are the interface between the stimulator/recorder 4 and the electrode 2. In the shown embodiment, the stimulator/recorder 4 comprises stimulation signal generator / recording electronics 6 for generating/recording a single signal S, a control logic 7 and a logic circuit 8. The control logic 7 provides inputs for the logic circuit 8. In turn, the logic circuit 8 provides the N channels to the respective N pads 3. These N channels represent the interface between the stimulator/recorder 4 and the electrode 2. When system 1 works as a stimulation system (single stage stimulation), logic circuit 8 may be implemented as a switch that allows distribution of stimulation current over the N channels. When system 1 works as a recording system, logic circuit 8 may be implemented as an amplifier with sequential sampling of the N channels. In other embodiments, the stimulation system can be implemented with N stages, or the recording system can be implemented with N separate amplifiers. In the shown embodiment, the N output channels 5 of the logic circuit 8 represent the output of the stimulator/recorder 4. Each pad 3 of the multi-pad electrode 2 must be connected to the stimulator/recorder 4 through a direct lead (or channel). Therefore, the number of pads 3 of the multi-pad electrode 2 is equal to the number of physical leads (channels) and limited to N. Increasing the number of pads 3 of the multi-pad electrode 2 in a system 1 like the one shown in Figure 1 would result in a linear increase of output channels of the stimulator/recorder 4 and an exponential increase of the overall system complexity.

Figure 2 shows a system 1000 for electrical stimulation and/or for electrical recording according to an embodiment of the invention. It comprises a multi-pad electrode 10 made of a flexible substrate. Multi-pad electrode 10 comprises a plurality of electrode pads 40. Multi-pad electrode 10 can be placed on the skin of an animal, such as a human being. For example, it may be placed on a skin portion of an arm, leg, chest or spinal area of the body of a user. The shape of the multi-pad electrode 10 may be adapted to the shape of the specific body part. The multi-pad electrode 10 may be placed on the user's skin such that at least some of the electrode pads 40 make electrical contact with the skin. The array of electrode pads 40 will then be in electrical contact, for example, with muscle tissue below the skin, being able to receive or transmit an electrical signal to the region of the muscle below the area of the skin occupied by the pads 40. Thus, the electrode pads 40 can provide through the skin a current, for example to the muscle tissue, in order to stimulate the muscle tissue. Activation/deactivation of selected electrode pads 40 is made through a digital circuit 25 printed on the flexible substrate. The digital circuit 25 may operate as a switching matrix or switching network. The digital circuit 25, also referred to as logic circuit, is the physical implementation of a logic layer or logic element. The digital circuit 25 is usually implemented by a plurality of switching elements (logic switching elements). In the multi-pad electrode 10 there is also an application specific layer, not shown in Figure 2, which is a connecting pathway between the stimulator/recorder 50 and the electrode pads 40. The application specific layer implements a plurality of application-specific switching elements, each of them being connected to a respective electrode pad 40. These switching elements allow the current flow between the signal generated by a stimulation/recording device 50 when the electrode works as stimulation electrode and an electrode pad 40 as selected by the digital circuit 25. And when the electrode works as recording electrode, the switching elements allow selection of one or more specific pads 40 in contact with the body part to be connected to the recording device.

As shown in Figure 2, system 1000 also includes a device for stimulation and/or recording (from now on, a stimulator/recorder or stimulation/recording device) 50. Unlike the ad-hoc made stimulator/recorder 4 of Figure 1, the stimulation/recording device 50 can be a commercial one, because it does not need to be specifically designed to work with the multi-pad electrode 10. The stimulation/recording device 50 is a conventional one and is out of the scope of the present invention. The stimulation/recording device 50 comprises a signal generator which determines the values of the parameters of the stimulation, such as amount of current and duration of the stimulation period. Typically, possible values of stimulation parameters may vary between minimum values and maximum values. In embodiments of the invention, the stimulation/recording device 50 may be a single channel stimulator or a single channel recorder, or both in the same device. A single channel stimulator provides a stimulation signal. A single channel recorder receives an electrophysiological signal. In the embodiment shown in Figure 2, a single channel stimulator/recorder 50 is used, which is directly connected to the multi-pad electrode 10, meaning that, when used as stimulation device, the generated stimulation signal is directly inputted to the multi-pad electrode 10, and when used as recording device, the electrophysiological signal captured by electrode 10 is directly provided to device 50. The direct connection between the stimulation/recording device 50 and the multi-pad electrode 10 is represented in Figure 2 with line 57.

A controller 60 is disposed between the stimulation/recording device 50 and the multi-pad electrode 10. Controller 60 generates control signals that will activate the desired pads 40 through the digital circuit 25. Controller 60 can control the operation of the digital circuit 25 in the multi-pad electrode 10 to route signals between the pads 40 of the multi-pad electrode 10 and the stimulation/recording device 50 and vice versa. Controller 60 includes a housing, inside of which a control logic unit 65 is provided, which provides the multi-pad electrode 10 with a plurality of control signals 17 that determine the state of switching elements that form the digital circuit 25. Controller 60 operates as an interface between the stimulation/recording device 50 and the multi-pad electrode 10. Controller 60 may include software embedded in processing means, such as a microprocessor. This software implements control logic 65. Control logic 65 provides the control signals for the logic circuits embedded in the multi-pad electrode 10 and thus controls which electrode pads 40 will be connected to the output of the stimulator/recorder 50. While the stimulation/recording signal goes directly respectively from the stimulator/recorder 50 to the multi-pad electrode 10 or from the multi-pad electrode 10 to the stimulator/recorder 50 through path 57, at the controller 60 control signals 17 are added to select the active pad or pads 40 to which the stimulation/recording signal goes.

In Figure 2, the digital circuit or logic circuit 25 embedded in the multi-pad electrode 10 is a switching logic circuit. As a matter of example, and with no limiting purpose, in Figure 2 it is implemented as a demultiplexing unit. The switching logic circuit 25 is embedded in the substrate of the multi-pad electrode 10, printed according to a printed electronics technique. Therefore, a number of physical leads equal to the number of electrode pads 40, for connecting the output of the stimulator/recorder 50 with individual electrode pads 40, is not required any more, as was the case in prior art systems for electrical stimulation and/or recording (see for example Figure 1). The switching logic circuit 25 can be implemented as a combination network or combination matrix (for example, a demultiplexing unit as in Figure 2, or a decoder unit). Alternatively, the switching logic circuit 25 can be implemented as a sequential network or sequential matrix. The logic circuit 25 allows to control all the electrode pads 40 with a limited number of input signals (or control signals). For example, when the logic circuit 25 is implemented as a demultiplexing unit, N input channels (inputs to the multi-pad electrode 10) can control 2^{N} electrode pads 40. One of the N input channels to the multi-pad electrode 10 is the stimulation/recording signal outputted by the stimulation/recording device 50 (path 57 in Figure 2) and the remaining N-1 input channels are N-1 digital control signals delivered by the control logic unit 65. It is therefore apparent that the proposed design allows for the use of a substantially larger number of pads 40 given a certain number of input signals to the multi-pad electrode 10. For example, implementing a combination network as the logic circuit 25 embedded (printed) in the substrate, as a demultiplexing unit, results in that the possible number of pads 40 is a power-of-2 function of the number of required physical leads (input channels to the multi-pad electrode 10). What is more, if the logic circuit 25 embedded (printed) in the substrate is implemented as a sequential network (instead of as a combination network), the number of physical leads (input channels to the multi-pad electrode 10) could be even further reduced, as explained in detail later.

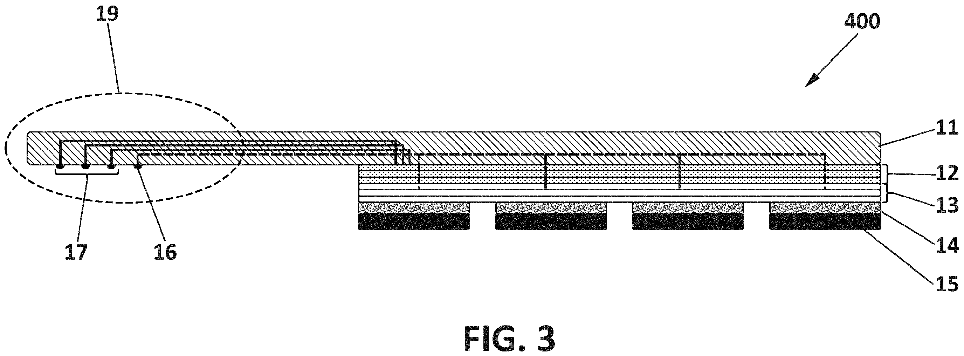

Figure 3 illustrates a cross-sectional view of a multi-pad electrode 400 according to an embodiment of the invention, that can be used in a system 1000 as shown in Figure 2. Multi-pad electrode 400 is made of a multilayer structure deposited on a flexible substrate or carrier 11, such as a flexible polymer film or a textile film. In embodiments of the invention, the flexible material of which the carrier 11 is made may be PET, TPU, PC, PS or another organic material with similar properties. In embodiments of the invention, the flexible material of which the carrier 11 is made may be a textile material. In this case, the textile carrier or specific parts of the textile carrier are preferably initially treated by deposition of a primer layer, on which the other layers of the electrode can be subsequently deposited. This primer layer is preferably based on TPU. Alternative materials such as PET, Polyimide and PDMS are possible but less preferred since they may introduce criticality with regard to screen-printable formulation and compatibility with subsequent printed electronic inks.

The logic circuit 25 (for example, a demultiplexing or decoder unit) and additional elements are embedded on a multilayer structure printed on the flexible substrate of which the multi-pad electrode 10 is made. In particular, the multilayer structure is implemented following a printing technique, such as screen printing, ink jet printing, aerosol jet printing, flexographic printing, offset printing, thermal transfer printing, or any combination thereof. In the schematic drawing of Figure 3, four different elements 12-15 printed on substrate or carrier 11 can be seen. In terms of operation of the multi-pad electrode 400, elements 12-14 are functional elements or functional layers, meaning that each of them has a different role in the multi-pad electrode 400, while element 11 (substrate) and element 15 (interface between electrode and user's skin) can be considered functionally idle. The different functional elements or layers 12-14 (and the idle ones 15, if any) are printed or deposited on the flexible substrate 11.

Each element (layer) 12-15 or group of elements is obtained by means of a printing technique, in which multiple passes (also referred to as layers or depositions) may be required in order to obtain each element or group of elements. Thus, the multi-pad electrode 400 is a multilayer structure (or multi-deposition structure) deposited on the substrate 11. The multilayer structure of electrode 400 is produced by deposition of different layers of printing materials on the flexible substrate 11, such as conductive materials, that form for example the leads and the electrode pads, dielectric materials, that isolate specific conductive parts and allow multi-layer printing, and electrochemically active materials, that act as semiconductors and allow formation of the digital circuit 25 of functional layer 12 and the switching elements of the application specific layer 13, onto the carrier or substrate 11. For example, the circuitry of element or layer 12 (and element 13, if they are printed simultaneously) may require more than 5 printing passes, layers or depositions, such as more than 10 printing passes. The required number of printing passes or depositions depends mainly on the complexity of the logic circuits and/or the application specific architectures.

Between the substrate or carrier 11 and the electrode pads 40 (layer 14), functional layers 12 and 13 implement circuitry, including electronic components, for connecting the electrode pads 40 to controller 60 and for controlling the activation/deactivation of the electrode pads 40. The required functional elements (functional layers) 12-14 are: a functional layer 12 implementing the digital circuit 25 (also called logic circuit); a functional layer 14 implementing the plurality of electrode pads 40; and an application-specific layer 13 comprising a plurality of switching elements, each of them connecting an output of the digital circuit 25 of functional layer 12 with an electrode pad 40. Functional layer 14 (electrode pads 40) is printed on top of layer 13. There is usually one electrode pad 40 per each switching element in layer 13. Each electrode pad 40 of layer 14 is preferably deposited -applying a printed electronics technique- on top of each corresponding switching element in layer 13. Finally, an electrode/skin interface layer 15 is optionally deposited on top of the electrode pads 40, that is to say, on top of layer 14. This interface layer 15 is recommended in order to achieve optimal fixing of the electrode pads 40 on the user's skin, while preventing the user to experience any discomfort or pain during use of the multi-pad electrode 400. Stable physical contact and proper energy exchange between the body and the electrode 400 is ensured by the electrode/skin interface layer 15. Interface layer 15 may also be used to achieve biocompatibility, in the event layer 14 does not ensure it. Electrode 400 also comprises a connector 19 allowing the stimulation/recording device 50 to interface the signal line 16 and the control lines 17 of the electrode 400. Connector 19 may be implemented in different ways, such as a plug, a flat cable connector or a pressure connector. Connector 19 has different connection interfaces with lines 16-17.

Logic circuit 25 of functional layer 12 is aimed at selecting (switching on or off) the electrode pads 40 to be active during use of the electrode 400. Functional layer 12 is implemented as a digital circuit 25 including switching elements (logic switching elements). Functional layer 12 is implemented, for example, as switching logic circuit 25 printed according to a printed electronics technique. Non-limiting examples of switching logic circuits are one or more combination networks, for example implemented as a demultiplexing unit or as a decoder unit; one or more sequential networks, for example implemented as a shift register; and a combination of one or more combination networks and one or more sequential networks. The switching logic circuit 25 is responsible for the activation/deactivation of pads. In a decoder unit, control bits are used to select which pad that should be active. The stimulation/recording signal is then transferred through a (application-specific) switching unit (for example an OECT) that is controlled by the decoder unit. In a demultiplexer circuit, address bits are used to select which pad is active, and then the stimulation/recording signal is transferred through the demultiplexer circuit as well, not only through the standalone (application-specific) switching element (for example OECT).

A decoder or a demultiplexing unit is less flexible because only one pad can be simultaneously selected, while an arbitrary number of pads can be activated by the shift register. Also, the shift register minimizes the number of control bits even more, as compared to the decoder or demultiplexing unit. However, the shift register is also much more complex in terms of number of components (for example transistors). The logic circuits 25 embedded in layer 12 can receive commands from controller 60 (in particular, from control logic 65). These commands are introduced to control lines 17 through corresponding connecting interfaces in connector 19. Control lines 17 are conductive paths transmitting the control signals to the appropriate inputs of the logic circuit 25 of layer 12. Then, the logic circuit 25 provides an appropriate signal to the switching elements forming layer 13. The conductive paths are printed when printing the rest of elements in layers 12 and 13. The conductive paths are all part of the multilayer structure. Through control lines 17 controller 60 (control logic 65) controls the logic circuits 25 (layer 12) embedded in the multi-pad electrode 10 and thus controls which electrode pads 40 will be connected to the output of the stimulator/recorder 50. Functional element or layer 13 allows the current flow between signal line 16 (path 57 in Figure 2) and each electrode pad 40 as selected by logic layer 12 (logic circuits embedded in element 12) according to a command introduced to control lines 17 delivered by the control logic 65. Signal line 16 is a conductive path transmitting either the signal from the stimulator/recorder 50 to the appropriate input of each of the switching elements in the application specific element or layer 13 when the electrode 400 is used as a stimulator, or the signal from a switching element (in turn from an electrode pad capturing the signal from the body) to be delivered to the stimulator/recorder 50 when the electrode 400 is used as a recorder. Conductive paths are printed when printing the rest of the multilayer structure (such as the functional elements 12-13) and are part of the multilayer structure. In other words, through signal line 16 the output of the stimulator/recorder 50 is delivered to specific switching elements in layer 13, in turn connected to respective pads 40 of element 14, or vice versa. Thus, functional layer 12 implements one or more logic circuits 25 that control propagation of N signals (inputted through connector 19) from the controller 60 to the electrode pads 40, and vice versa. In turn, application specific element 13 implements a plurality of switching elements configured to connect the digital circuit 25 (of layer 12) to each electrode pad 40 of functional layer 14. For example, when the logic circuit 12 is implemented as a demultiplexing unit, each switching element of layer 13 connects one output of the demultiplexing unit (functional layer 12) with one electrode pad 40 (functional layer 14). There is one switching element per electrode pad. Thus, connection of two different outputs of the functional element 12 to a single electrode pad 40 is prevented. Electrode pads 40 can be independently activated/deactivated by the digital circuit 25 (logic element 12) through respective switching elements of layer 13.

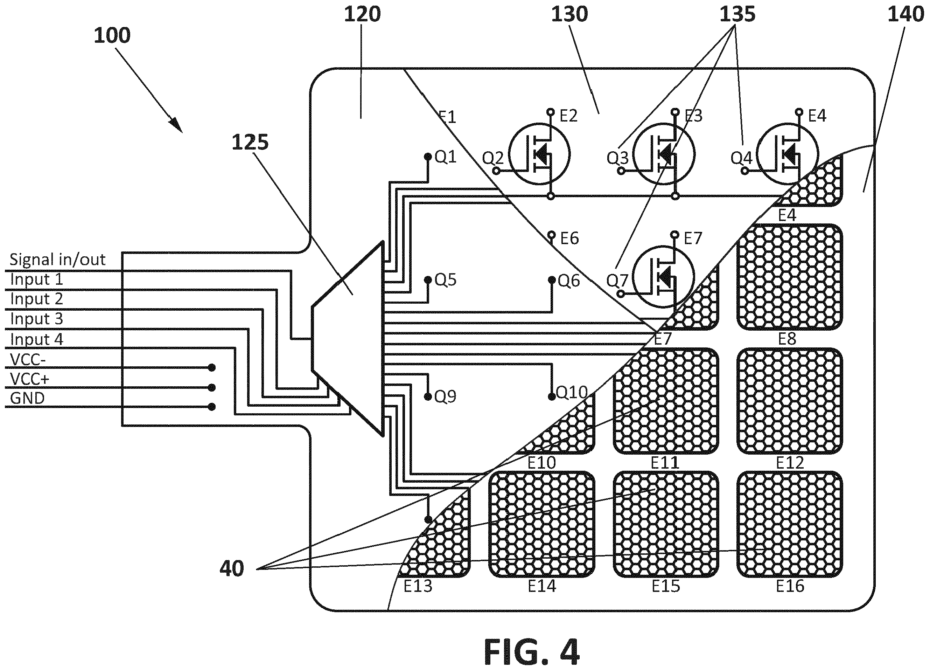

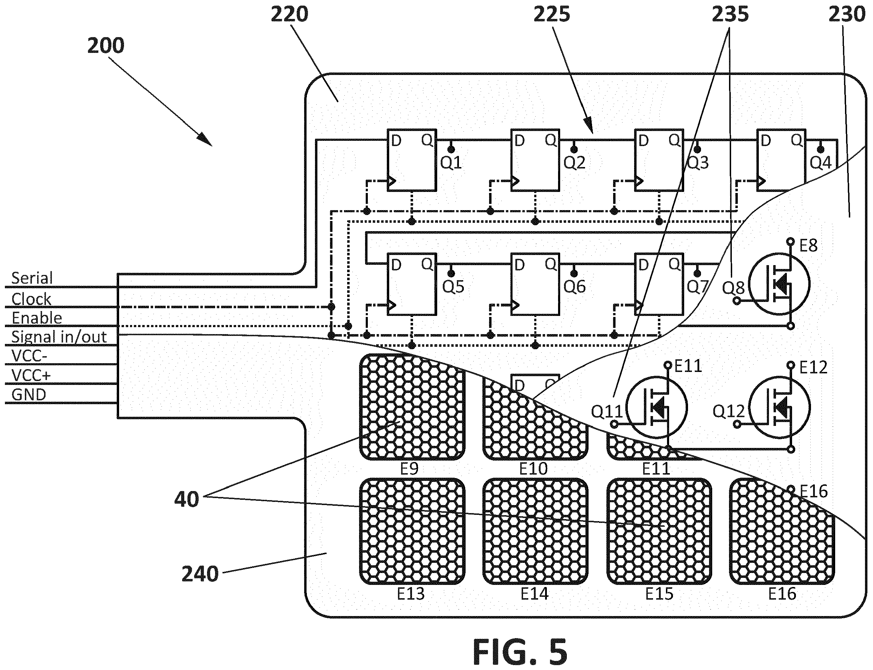

The selection of the specific switching elements (application-specific switching elements) in application-specific layer 13 is made by the digital circuit 25 (functional layer 12). The switching elements in application-specific layer 13 are application-specific switching devices needed for the transmission of different types of signals (stimulation signals or recording signals) to/from corresponding electrode pads. Stimulation signals are usually defined in current, while recording signals are defined in voltage. For example, stimulation signals have currents in the range of milliamperes (mA) generated at tens of volts (V); and recording signals are signals having voltage in the range of microvolts (µV). In sum, regarding the application-specific layer 13, in case of stimulation, application-specific switching elements are switches for passing large stimulation currents, while in case of recording, application-specific switching elements are precise switches that transmit the EMG signals (microvolts, µV) and may even provide amplification. In the proposed embodiments, when the electrode is configured or used for stimulation, the application-specific element may be comprised of transistor elements (switches), such as OECTs, designed for large current throughput, while when the electrode is configured or used for recording, the application-specific element 13 may be comprised of transistor elements, such as OECTs, arranged in a configuration that amplifies and/or blocks/transfers the signal received from the respective pads 40 of element 14. Logic layer or element 12 and application specific layer or element 13 are explained in more detail with reference to Figures 4 and 5.

In embodiments of the invention, an electrode 10, 400 is designed only for recording. In embodiments of the invention, an electrode 10, 400 is designed only for stimulation. In embodiments of the invention, a single electrode 10, 400 is designed for both stimulation and recording. In this case, different designs can be implemented. In one embodiment, the electrode has a first plurality of electrode pads for stimulation and a second plurality of pads for recording. This essentially implies having two electrodes on one substrate. In another embodiment, only a plurality of electrode pads is required, each pad being used for recording and stimulation. In this case, the application specific layer 13 is actually a general purpose one that passes an unconditioned signal and can sustain high current throughput.

In embodiments of the invention, logic circuit 25 of layer 12 is implemented with transistors. Non-limiting examples of transistor technologies that may be used are MOS (such as NMOS, PMOS or CMOS), field effect transistors (FET) and organic electrochemical transistors (OECT). In a preferred embodiment, the logic circuits 25 of layer 12 are based on OECTs. In this case, the logic circuits -switching logic circuits- are multi-layered OECT-based logic circuits and are made from a plurality of depositions of conductive, dielectric, electrolytic and electrochemically active organic materials. The total number of depositions and the circuit topology will depend on the number of pads 40 to be controlled and the design of the switching logic circuits. As a matter of example, with no limitation purpose, two different designs are shown in Figures 4 and 5.

In embodiments of the invention, the switching elements of application-specific layer 13 are implemented with transistors. In other words, the switching elements of the application-specific element 13 may be transistors configured as switches. Non-limiting examples of transistors that may be used are MOS (such as NMOS, PMOS or CMOS), field effect transistors (FET) and organic electrochemical transistors (OECT). In a preferred embodiment, application-specific layer 13 is based on OECTs. In this case, the multilayer structure of application-specific layer 13 is made from a plurality of depositions of conductive, dielectric, electrolytic and electrochemically active organic materials, forming a plurality of OECTs. The total number of depositions and the circuit topology will depend on the selected application, such as stimulation or recording (or both), and on the requirements of each application, such as a required gain for recording, a required current throughput for stimulation and a required OECT ON/OFF ratio to properly block/transfer the signal when required.

Layers 12-13 can be printed at the same time (simultaneously, in the same printing process), in which case the same transistor technology must be used for both functional layers 12-13. In preferred embodiments, layers 12-13 are manufactured simultaneously, in the same printing process, since the materials required for the respective layer are identical.

Preferably, OECT are used, because of the possibility they offer to use printing techniques for the manufacturing of the multi-pad electrodes on top of flexible substrates. For example, OECTs can be manufactured on flexible substrates by using only printing techniques, such as screen printing. Besides, OECTs can deliver high current levels or throughput; because the charge transport occurs in the bulk of the channel material. They are operated at low voltages (about 1 V). There is no particular requirement on switching time, since we only need to address them once and then stimulation/recording goes on for a certain amount of time.

Although this is more important for the switching elements forming the application specific layer 13 than for the logic circuit 25 of functional layer 12, since layer 12 and layer 13 can be printed at the same time, OECTs are preferably used. Furthermore, OECTs are also relatively more biocompatible as compared to transistors based on inorganic materials. They are operating at low voltages, and may also 'sense' low voltages, hence, they are compatible with bioelectronic signals, and they are also used as transducers between ionic and electronic signals, and vice versa. From a printing perspective, layer 12 is more complex to create, due to a few more printed layers (depositions). But they can be manufactured at the same time, in the same process. On the other hand, one reason to separate them into logic layer 12 and application-specific layer 13 is to allow for usage of OECTs only in one of them, or in both of them. In case of only in one of them, it allows for usage of any other transistor technology in the other layer.

As a matter of example, with no limiting purpose, the deposition/printing of layers 12-13 on a flexible substrate may be made as follows: Required layers: silver, PEDOT:PSS (semiconductor), electrolyte, PEDOT:PSS (again, as the gate electrodes). Optional layers: dielectric (also referred to as insulator, to define the area of the subsequently deposited electrolyte), another insulator on top of the gate electrode (for mechanical protection). Additionally, for layer 12 (circuits), wire crossings are most often needed, hence, another silver layer is then needed. To also establish layer 14, yet another insulating layer and yet another silver layer are needed. But this can also be deposited in the same printing process, in which case the number of passes increases.

Figure 4 illustrates a multi-pad electrode 100 according to an embodiment of the invention. Electrode 100 is schematically shown in functional layers 12-14 (referred to as 120-140, respectively, in Figure 4), but in view of the multilayer structure of the electrode that has been described, a person skilled in the art will understand that the functional layers 12-13 (120-130) of the printed structure may be mixed, for example when they have been printed in the same printing process. In Figure 4, functional layer 140, which in the scheme covers functional layer 130, has been partially removed in order to show functional layer 130. In turn, functional layer 130, which in the scheme covers functional layer 120, has been partially removed in order to show functional layer 120. In the shown embodiment, layer 140 comprises 16 pads (such as square pads) labelled E1 to E16, arranged in a 4x4 matrix (distributed in four rows of four pads per row). In electrode 100, the logic circuits 125 of functional layer 120 is implemented as a combination network, in this case, a demultiplexer. Alternatively, it may be a decoder. The main difference between a decoder and a demultiplexer is that the decoder selects the pads, and the pulse signal is then only transferred through the selected pad, but the pulse signal does not have to pass through the decoder circuit. A demultiplexer also selects the pads, but the pulse signal is also transferred through the demultiplexer circuit.

The logic circuits 125 of logic layer 120 are based on OECTs. The combination network implemented by the logic circuits 125 is a 4-to-16 demultiplexer, meaning that four control signals (labelled as Input_1 to Input_4 (Input-N in a more general approach) in Figure 4) are enough to control de selection (activation/deactivation) of the 16 pads 40. Layer 120 controls the activation of the switching elements 135 (in this case, also OECTs) in the application specific layer 130, allowing the selection of one of the 16 pads 40. Demultiplexer 125 allows the "signal in/out" (travelling in path 57 of Figure 2 or 17 in Figure 3) to propagate to one of the 16 pads 40, based on the 4 input signals "lnput_1", "lnput_2", "Input_3" and "Input_4" as provided by control logic 65. As can be seen, each of the 16 outputs of the demultiplexer 125 connects to a corresponding switching element 135 of layer 130, which in this case is a OECT (labelled Q1 to Q16 in Figure 4). The application specific layer 130 (its switching elements 135) permits the propagation of the signal "signal in/out" from the connector 19 (in fact, from the stimulation/recording device 50) to the electrode pads 40 when the electrode 100 is used in a stimulation application or vice versa (the propagation of the "signal in/out" from the electrode pads 40 to the connector 19 (in fact, to the stimulation/recording device 50) when the electrode 100 is used in a recording application. In other words, when the electrode 100 operates as a stimulator, the "signal in/out" may be a "pulse in" signal provided by stimulator/recorder 50, and when the electrode 100 operates as a recorder, the "signal in/out" may be an output signal as captured by the active pad or pads 40. The logic circuit 125 selects (using a signal of for example 1V) which switching element 135 to turn on, so that the "signal in/out" (a signal of for example 100 V in case of stimulation; a signal of for example 100 µV in case of recording) passes through that switching element. For the digital circuit 125 to operate, additional lines for Vcc+, Vcc-, GND are needed, leading to a total number of 8 lines for control of 16 pads. So, disregarding these 3 lines (Vcc+, Vcc-, GND), with N lines (one "signal in/out" from/to the stimulating/recording device 50 and N-1 control lines from the control logic 65), 2^{N-1} pads can be controlled. Or, in other words, if the "signal in/out" is excluded, with N lines (Input_1 to Input_4) from the control logic 65, activation/deactivation of one of 2^{N} electrode pads 140 can be made.

In a stimulation application, OECTs 135 in layer 130 are designed to allow high current throughput when applying the "signal in/out". In a recording application, OECTs 135 in layer 130 are designed to amplify the input signal in layer 13, before it is connected to the "signal in/out" connection 16 (see Figure 3). In this text, the term "amplify" should be understood as to obtain sufficiently high ON/OFF ratio, to either block or transfer the signals.

Figure 5 illustrates a multi-pad electrode 200 according to another embodiment of the invention. Like electrode 100, electrode 200 is schematically shown in functional layers 220-240, but in view of the multilayer structure of the electrode that has been described, a person skilled in the art will understand that the functional layers 220-230 of the printed structure may be mixed, for example when they have been printed in the same printing process. Like in Figure 4, functional layer 240, which in the scheme covers layer 230, has been partially removed in order to show functional layer 230. In turn, functional layer 230, which in the scheme covers functional layer 220, has been partially removed in order to show functional layer 220. Like in Figure 4, layer 240 comprises 16 pads (such as square pads), labelled E1 to E16, arranged in a 4x4 matrix (distributed in four rows of four pads per row). In electrode 200, the logic circuits 225 of functional layer 220 is implemented as a sequential network, such as a shift register 225, in this case a 16-bit shift register.

The logic circuits 225 of logic layer 220 are based on OECTs. This layer 220 controls the activation of the switching elements 235 (in this case, also OECTs) in the application specific layer 230, allowing for the simultaneous selection of 0 to M pads, M being the total number of electrode pads 40 in layer 240. In the shown embodiment, M = 16. Shift register 225 allows the propagation of "signal in/out" (travelling in path 57 of Figure 2 or 17 in Figure 3) to the (0 to M) electrode pads 40 in a stimulation application, or vice versa (from the (0 to M) electrode pads 40 to path 57 of Figure 2 or 17 in Figure 3) in a recording application, based on a single control line (referred to as "serial" in Figure 5) and a clock line (referred to as "clock" in Figure 5). The logic circuit 225 selects (using a signal of for example 1V) which switching element 235 to turn on, so that the "signal in/out" (a signal of for example 100 V in case of stimulation; a signal of for example 100 µV in case of recording) passes through that switching element. The advantage of a solution based on a sequential network (such as shift register circuit) with respect to a combination network (such as a decoder or a demultiplexer) is that the electrode pad selection can be achieved according to any arbitrary pattern, since each electrode pad 40 in layer 240 can be independently activated or inactivated. For the digital circuit (shift register 225) to operate, additional lines for Vcc+, Vcc-, GND and ENABLE are needed, leading to a total number of 7 lines for control of 16 pads. In this particular approach, any number of pads could be controlled by the same 7 lines, completely removing the dependency between the number of pads and the number of physical leads. However, this is reflected in the complexity of the circuit and the number of operations needed to set the desired state. This may impose a limitation in time demands when performing switching, which might be critical for certain applications requiring dynamic change of electrode configuration. In a stimulation application, OECTs 235 in layer 230 are designed to allow high current throughput when applying the "signal in/out". In a recording application, OECTs 235 in layer 230 are designed to amplify the input signal in layer 13 before it is connected to the "signal in/out" connection 16 (see Figure 3). As before, the term "amplify" should be understood as to obtain sufficiently high ON/OFF ratio, to either block or transfer the signals.

The proposed multi-pad electrode 1000, 400, 100, 200 can be used as a wearable biomedical electrode. The multi-pad electrode can be designed for bio-signal recording applications, in which case the switching elements, such as application specific OECTs, are specifically designed and configured to provide a simple switching (ability to switch between a high and a low impedance state) and/or localized amplification of an input signal. The multi-pad electrode can be designed for stimulation applications, in which case the switching elements, such as application specific OECTs, are specifically designed and configured to provide a simple switching (ability to switch between a high and a low impedance state) as well as to allow high voltages and high current throughput (when switched to a low impedance state) provided by the output signal.

Turning back to Figure 2, when the stimulation/recording device 50 is configured as stimulator, that is to say, configured to provide stimulating electrical signals to the multi-pad electrode 10, 100, 200, 400, the stimulating electrical signal is preferably provided as a biphasic electrical signal, wherein the negative polarity of said biphasic electrical signal precedes the positive polarity of said biphasic electrical signal. For example, a pulse train is used, having pulse widths between 100 and 1000 µs for each phase; and having frequency between 10 and 200 Hz. Other frequencies may be used instead. Inventors have observed that this configuration permits to obtain the highest performance. The reason for the improved performance is that the transistors (preferably OECTs) have a better chance to remain in its high impedance state if the negative pulse is provided before the positive pulse, hence, less leakage current in deactivated electrode pads.

In this text, the term "comprises" and its derivations (such as "comprising", etc.) should not be understood in an excluding sense, that is, these terms should not be interpreted as excluding the possibility that what is described and defined may include further elements, steps, etc.

In the context of the present disclosure, the term "approximately" and terms of its family (such as "approximate", etc.) should be understood as indicating values very near to those which accompany the aforementioned term. That is to say, a deviation within reasonable limits from an exact value should be accepted, because a skilled person in the art will understand that such a deviation from the values indicated is inevitable due to measurement inaccuracies, etc. The same applies to the terms "about", "around" and "substantially".

The invention is obviously not limited to the specific embodiment(s) described herein, but also encompasses any variations that may be considered by any person skilled in the art (for example, as regards the choice of materials, dimensions, components, configuration, etc.), within the general scope of the invention as defined in the claims.

## Claims

1. - A multi-pad electrode (10, 100, 200, 400), comprising a flexible substrate (11) having a multilayer structure printed thereon, the multilayer structure comprising:
a logic circuit (25, 125, 225),
a plurality of switching elements (135, 235) configured for stimulation or recording, and
a plurality of electrode pads (40), each electrode pad (40) being in contact with a respective switching element (135, 235),
wherein each switching element (135, 235) is configured to connect the logic circuit (25, 125, 225) to a respective electrode pad (40), the logic circuit (25, 125, 225) being configured to activate or deactivate each electrode pad (40, by controlling propagation of an electrical signal (signal in/out) to each switching element (135, 235) and therefore to each electrode pad (40) or vice versa.

2. - The multi-pad electrode (10, 100, 200, 400) of claim 1, further comprising an interface layer (15) in contact with the plurality of electrode pads (40), the interface layer (15) being an interface configured to ensure stable physical contact between the electrode pads (40) and the skin of the person wearing the multi-pad electrode.

3. - The multi-pad electrode (10, 100, 200, 400) of any one of claims 1-2, wherein the flexible substrate (11) is made of a flexible polymer film.

4. - The multi-pad electrode (10, 100, 200, 400) of any one of claims 1-2, wherein the flexible substrate (11) is made of a textile material.

5. - The multi-pad electrode (10, 100, 200, 400) of claim 4, wherein the textile material of the flexible substrate (11) comprises an elastomeric material deposited thereon.

6. - The multi-pad electrode (10, 100, 200, 400) of claim 5, wherein the elastomeric material deposited on the textile material is a thermoplastic polyurethane (TPU).

7. - The multi-pad electrode (10, 100, 400) of any one of claims 1-6, wherein the logic circuit (25, 125), is either a demultiplexing unit or a decoder unit configured to allow activation or deactivation of one of 2^{N} electrode pads (40) based on N control input signals (Input_1 - Input_N).

8. - The multi-pad electrode (10, 200, 400) of any one of claims 1-6, wherein the logic circuit (25, 225) is a shift register configured to allow simultaneous selection of from zero to M electrode pads (40) based on clock (Clock) and data (Serial) signal inputs, M being the total number of electrode pads (40).

9. - The multi-pad electrode (10, 400) of any one of claims 1-6, wherein the logic circuit (25) comprises at least one demultiplexing unit or decoder unit (125) and at least one shift register (225) configured to allow simultaneous selection of an arbitrary number of electrode pads (40) based on the combination of control, clock and data input signals.

10. - The multi-pad electrode (10, 400) of claim 9, wherein the at least one shift register (225) is configured to select a set of electrode pads, thereby forming virtual electrodes, while the at least one demultiplexing unit or decoder unit (125) is configured to dynamically switch between previously defined virtual electrodes.

11. - The multi-pad electrode (10, 100, 200, 400) of any one of claims 1-10, wherein the plurality of switching elements (135, 235) is made of organic electrochemical transistors.

12. - The multi-pad electrode (10, 100, 200, 400) of any one of claims 1-11, wherein the logic circuit (25, 125, 225) is based on organic electrochemical transistors.

13. - The multi-pad electrode (10, 100, 200, 400) of any one of claims 1-12, wherein the multilayer structure printed on the flexible substrate (11) is printed by one of the following printing techniques: screen printing, ink jet printing, aerosol jet printing, flexographic printing, offset printing, thermal transfer printing, or any combination thereof.

14. - A system for electrical stimulation and/or recording (1), comprising:
a multi-pad electrode (10, 100, 200, 400) according to any one of the preceding claims,
a stimulation/recording device (50), configured to provide stimulating electrical signals to the multi-pad electrode (10, 100, 200, 400), or recording of electrical signals from active electrode pads,
a controller (60) configured to control the propagation of electrical signals from/to the stimulation/recording device (50) to/from the electrode pads (40) of the multi-pad electrode (10, 100, 200, 400).

15. The system of claim 14, wherein, when it is configured to provide stimulating electrical signals to the multi-pad electrode (10, 100, 200, 400), the stimulating electrical signal is provided as a biphasic electrical signal, wherein the negative polarity of said biphasic electrical signal precedes the positive polarity of said biphasic electrical signal.
